(19) **Europäisches Patentamt**
**European Patent Office**
**Office européen des brevets**

(11) **EP 2 124 826 B2**

(12) **NEW EUROPEAN PATENT SPECIFICATION**
After opposition procedure

(45) Date of publication and mention
of the opposition decision:
**23.09.2020 Bulletin 2020/39**

(45) Mention of the grant of the patent:
**02.07.2014 Bulletin 2014/27**

(21) Application number: **08725659.0**

(22) Date of filing: **15.02.2008**

(51) Int Cl.:
***A61F 2/24*** *(2006.01)* ***A61F 2/82*** *(2013.01)*

(86) International application number:
**PCT/US2008/002049**

(87) International publication number:
**WO 2008/100599 (21.08.2008 Gazette 2008/34)**

(54) **MULTI-LAYERED STENTS**

MEHRSCHICHTIGE STENTS

ENDOPROTHÈSES VASCULAIRES MULTICOUCHES

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **15.02.2007 US 901582 P**

(43) Date of publication of application:
**02.12.2009 Bulletin 2009/49**

(73) Proprietor: **Medtronic, Inc.**
**Minneapolis, MN 55432-5604 (US)**

(72) Inventors:
• **BONHOEFFER, Philipp**
**London NW1 9XP (GB)**
• **SCHIEVANO, Silvia**
**London WC1H 8HX (GB)**
• **RYAN, Timothy, R.**
**Shorewood, Minnesota 55331 (US)**
• **LASKE, Timothy, G.**
**Shoreview, Minnesota 55126 (US)**

(74) Representative: **Zimmermann & Partner**
**Patentanwälte mbB**
**Postfach 330 920**
**80069 München (DE)**

(56) References cited:
WO-A-03/099168 WO-A-2004/016201
WO-A-2004/091454 WO-A-2006/014347
WO-A1-98/29057 WO-A1-2004/021929
WO-A2-03/099168 US-A- 5 411 552
US-A1- 2004 210 306 US-A1- 2005 278 017
US-A1- 2005 278 017 US-B1- 6 425 916
US-B2- 7 018 406

• **Boudjemline et al: "Steps Toward Percutaneous
Aortic Valve Replacement", Circulation, vol. 105,
2002,**
• **Boudjemline et al: "Percutaneous pulmonary
valve replacement in a large right ventricular
outflow tract", Journal of the American College
of Cardiology, vol. 43, no. 6, 2004,**

EP 2 124 826 B2

## Description

### Technical Field

The present invention relates to stents used in the treatment of cardiac and venous valve disease. More particularly, it relates to minimally invasive and percutaneous implantation of stents in the treatment of cardiac and venous valve disease.

### Background

Stents are commonly used for treatment of a wide variety of medical conditions; Stent fractures are a phenomenon to be avoided, particularly when such fractures are so numerous and/or severe that they disrupt or destroy the functioning of the stent. For example, stent fracture is a recognized complication that can occur following stent implantation in cardiovascular applications, which can result in disruption of the normal functioning of the heart. Certain factors and combinations of factors can increase the chances of a stent fracture occurring, such as choosing a stent wire size that is not appropriate for a stent that is subjected to relatively severe structural loading conditions, the application of high stresses, and other factors. Thus, a number of different stent configurations and designs have been proposed for certain stent applications in an attempt to eliminate or reduce the occurrence of stent fracture, with the goal of enhancing stent performance and durability.

In the field of valved stent technology, there has been an increased level of interest in minimally invasive and percutaneous replacement of cardiac valves, including pulmonary valves, aortic valves, and mitral valves. However, the stresses encountered by such products can be extreme. This can result in failure of some stents, as is described in U.S. Patent Application Publication No. 2005/0251251. This publication also recognizes the problems caused by stent recoil in these relatively weak stents that do not allow the stents to be forcefully imbedded into an aortic annulus and the risks of massive regurgitation through the spaces between frame wires. The wires used for such stents can also be more prone to fracture than the thicker wires used in other stent implantation applications.

Designers of transcatheter delivered heart valves face additional problems such as paravalvular leakage, thrombus formation, eblolization, infection, sizing, valve degeneration, pannus formation, migration, interference with coronary function, and ischemia.

In an exemplary context of pulmonary valve replacement, U.S. Patent Application Publication Nos. 2003/0199971 A1 and 2003/0199963 A1, both filed by Tower, et al., describe a valved segment of bovine jugular vein, mounted within an expandable stent, for use as a replacement pulmonary valve. The replacement valve is mounted on a balloon catheter and delivered percutaneously via the vascular system to the location of the failed pulmonary valve and expanded by the balloon to compress the native valve leaflets against the right ventricular outflow tract, anchoring and sealing the replacement valve. As described in the articles: "Percutaneous insertion of the pulmonary valve", Bonhoeffer, et al., Journal of the American College of Cardiology 2002; 39(10): 1664 - 1669; "Transcatheter Replacement of a Bovine Valve in Pulmonary Position", Bonhoeffer, et al., Circulation 2000; 102: 813 - 816; and "Percutaneous replacement of pulmonary valve in a right-ventricle to pulmonary-artery prosthetic conduit with valve dysfunction", Bonhoeffer, et al., Lancet 2000; 356 (9239): 1403-1405. A replacement pulmonary valve may be implanted to replace native pulmonary valves or prosthetic pulmonary valves located in valved conduits, such as in the treatment of right ventricular outflow tract dysfunction, for example. A number of implantable stents, many of which are expandable and compressible for insertion into a heart valve using percutaneous delivery methods and systems, are also described, for example, in U.S. Patent Nos. 6,425,916 (Garrison) and 7,060,089 (Ley et al.); U.S. Patent Application Publication Nos. 2005/0075725 (Rowe), 2005/0251251 (Cribier), 2006/0271166 (Thill et al.), 2006/0276874 (Wilson et al.), and 2007/0213813 (Von Segesser et al.); and PCT International Publication Nos. WO 2007/053243 (Salahieh et al.), WO 2006/054107 (Bonhoeffer), and WO 2007/081820 (Nugent et al.).

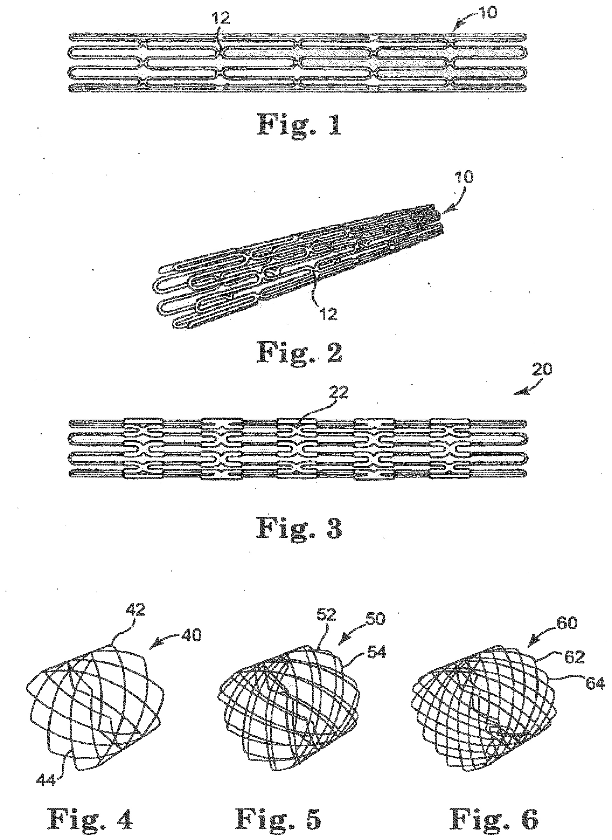

Percutaneous pulmonary valve implantation generally involves transcatheter placement of a valved stent within an existing degenerated valve or conduit, and can often provide excellent hemodynamic results, including relief of right ventricular outflow tract obstruction, significant reduction in pulmonary regurgitation, right ventricular pressure and right ventricular outflow tract gradient, and improvement in exercise tolerance, as are described in the articles: "Percutaneous pulmonary valve implantation in humans: results in 59 consecutive patients", Khambadkone, et al., Circulation 2005; 112(8): 1189-1197; and "Physiological and clinical consequences of relief of right ventricular outflow tract obstruction late after repair of congenital heart defects", Coats, et al., Circulation 2006; 113(17): 2037-2044. Some of the first stents used for percutaneous pulmonary valve implantation were created by a platinum/iridium wire, which was formed into a zigzag shaped pattern, with the individual segments being joined together at the crowns by welding of the platinum. Exemplary areas of platinum welds are shown as welds 12 of a stent 10 in Figures 1 and 2. One disadvantage of these stents is that the platinum welds at the strut intersections, along with other areas of the stents, were prone to fracture during or after implantation into a patient. This was due in part to the relatively severe structural loading conditions placed on the stents through the stent compression and expansion processes used for percutaneous implantation, along with the design of the stents used in these processes. As discussed above, such fractures can be problematic, particularly as the desirability for more long-term stent durability increases.

One proposed way of minimizing stent fracture at the welds was to use a gold brazing process to reinforce the crowns of the stent. An exemplary version of such a stent is illustrated with multiple gold reinforcement areas 22 of a stent 20 in Figure 3. However, even with these gold-reinforced stents, some stent fractures were still found to occur. In particular, while the gold-reinforced stents did not typically exhibit fractures at strut intersections, as with stents having platinum welds, gold-reinforced stents still showed fractures at areas adjacent to or spaced from the strut intersections. It was found that these fractures occurred during the process of crimping the stent onto a delivery system balloon, after the balloon dilation process, after implantation of a second percutaneous valve, or even spontaneously.

Another way that was proposed to overcome the risks associated with fractured implanted stents involves interventional management of the stent fracture by repeat percutaneous pulmonary valve implantation to provide stabilization of the fractured parts. This technique is sometimes referred to as a "stent-in-stent" technique, which involves implanting a new stent in the area of the previously implanted fractured stent. The feasibility of stent-in-stent implantation has been demonstrated with different stents for a variety of indications in congenital heart disease, such as is described in the articles: "Prolongation of RV-PA conduit life span by percutaneous stent implantation. Intermediate Term Results", Powell, et al., Circulation 1995; 92(11): 3282-3288; "Longitudinal stent fracture 11 months after implantation in the left pulmonary artery and successful management by a stent-in-stent maneuver", Knirsch, et al., Catheterization and Cardiovascular Interventions 2003; 58: 116-118; "Implantation of endovascular stents for the obstructive right ventricular outflow tract", Sugiyama, et al., Heart 2005; 91(8): 1058-1063; and "Stress stent fracture: Is stent angioplasty really a safe therapeutic option in native aortic coarctation?", Carrozza, et al, International Journal of Cardiology 2006; 113(1): 127-128. Although this stent-in-stent approach can be helpful in overcoming stent fracture concerns, there is a continued desire to provide improved stents that can be implanted in a simple minimally invasive and percutaneous manner, while minimizing the risks associated with stent fracture. Such improved stents may be particularly useful in more challenging loading conditions, such for use in the areas of the aortic and mitral valves, and for treating medical conditions that have increasing long-term durability requirements.

## Summary

The present invention is defined by the appended claims. The present invention is particularly directed to improvements in valves that can be delivered in a minimally invasive and percutaneous manner, which are most preferably useful for the pulmonary valve position, although the valves can also be useful for the aortic valve position. In addition, the stents and related concepts of the invention may also be useful in other types of medical applications, including replacement of other heart valves (e.g., mitral valves) and peripheral venous valves. Further, the stents and valves of the invention can be used in implantations that are performed in more invasive surgical procedures than those involved in percutaneous valve delivery. The valves of the invention include stents that are multi-layered that are produced by combining stents of various materials and designs to take advantage of their different mechanical properties, reinforce the prosthesis (i.e, meet radial force requirements), and avoid or minimize the occurrences of fractures. The configuration and components of the elements of the stents can further be customized to provide a valve that allows for a desired amount of tissue ingrowth and minimizes paravalvular leakage.

The multi-layered valves include at least an inner stent and an outer stent, where the inner stent is allowed to move substantially independently of the outer stent, although it is understood that the multi-layered devices of the invention can include more than two stents such that the description of devices having inner and outer stents herein is intended to include additional stents inside, outside, and/or between the inner and outer stents, when desired. In one exemplary embodiment, a single device can provide the advantages of both relatively rigid and relatively flexible portions, where a more rigid outer stent provides strength to the device and a more flexible inner stent can advantageously absorb and adapt to stresses and strains caused by flexure of the device in operation. At the same time, the outer stent can protect the inner stent from being subjected to certain stresses. For another example, a mere rigid outer stent can help the device to be successfully implanted in an irregularly shaped location, since a relatively rigid stent can force an orifice to conform more closely to the shape of the stent, while the more flexible inner stent is allowed to flex independently. In another example not falling under claim 1, the device can be include a more flexible outer stent that can better conform to the anatomy of the patient and a more rigid inner stent that provides a stable base for supporting a leaflet structure. Thus, the materials selected for each of the stents, in combination with the specific features and designs chosen for each of the stents, can provide device performance that cannot be achieved by single-layered stent and can allow for the use of materials that have material properties that may not otherwise be useful in a single-layered stent.

In at least some embodiments of the invention, multiple stents are attached to each other prior to implantation in a patient, such that a multi-layered stent is delivered in a single procedure, with the multi-layered stent being delivered as a single unit. The stents may be attached to each other in a wide variety of ways, depending on the configurations and materials of each of the stents. For example, the stents may be attached to each other by welding, suturing, bending or folding of components relative to each other, or with the use of connecting mechanisms such as clips, barbs, hooks, and the like. Alternatively, the stents may be attracted to each other or held together with a frictional type of force. In any case, the number and locations of the attachment points can vary, depending on the amount of relative movement between the stents that is desired.

Each of the stents in the multi-stent configurations of the present invention are different from each other with respect to a number of features. Each of the stents have different thicknesses, stiffnesses, geometries, lengths, or other material properties. For another example, one of the stents can be provided with larger openings (i.e., a more open wire density) than the openings of another stent in the same structure, where the relative sizes of these openings can encourage or inhibit tissue ingrowth, depending on the desired stent performance.

A multi-stent configuration in some embodiments will include two stents, but in other embodiments, more than two stents can be used. One or more stents or portions of stents can be bioabsorbable. Both stents may be self-expanding. With either of these stent structures that include multiple stents with similar expansion characteristics, both stents will expand or be forced to expand in a substantially simultaneous manner. Alternatively, one stent or part of one of the stents can be balloon expandable while an inner stent is self-expanding. In one particular exemplary embodiment, an inner stent of a device is constructed from a shape-memory type of material (e.g., Nitinol) so that it is self-expandable, while the outer stent of the same device can be expandable by the application of outward radial forces, such as can be provided by the balloon of a delivery system. In another exemplary embodiment, the outer stent of a device is constructed from a shape-memory type of material so that it will expand upon initial deployment of the multi-stent device.

One or more of the stents of a multi-stent structure can include a complete or partial covering, if desired. In particular, a covering or partial covering can be provided on the outer surface of the outermost stent of a multi-stent structure, and/or on the inside surface of the innermost stent of a multi-stent structure, and/or in between any or all layers of a multi-layer stent structure. Such a covering can be provided to impart some degree of fluid permeability or impermeability and/or configured to promote or limit tissue ingrowth for the purpose of sealing and or anchoring the stent structure. The covering can further be provided to carry and and/or deliver drugs and/or growth factors to limit or prevent restenosis, endocarditis, platelet pannus, infection, and/or thrombus. The covering may be made at least partially of a fabric, tissue, metallic film, and/or a polymeric material.

## Brief Description of the Drawings

The present invention will be further explained with reference to the appended Figures, wherein like structure is referred to by like numerals throughout the several views, and wherein:

Figure 1 is a front view of a stent including platinum welds between various adjacent struts;
Figure 2 is a perspective view of the stent of Figure 1;
Figure 3 is a front view of a stent of the type illustrated in Figure 1, and further including multiple reinforcement areas;
Figure 4 is a perspective view of one example useful for understanding the invention of a multiple layer stent in accordance with the invention;
Figure 5 is a perspective view of another example useful for understanding the invention of a multiple layer stent, with the two stents rotated relative to each other;
Figure 6 is a perspective view of another example useful for understanding the invention of a multiple layer stent, with the two stents further rotated relative to each other:
Figures 7-9 are Von Mises stress maps of three stents (a PL stent, a PL-AU stent, and a $PL_{1/2}$ stent) at the end of a simulated balloon inflation and including an enlarged view of a portion of the stent to better illustrate the stress concentrations in that portion;
Figures 10-12 are Von Mises stress maps of the three stents of Figures 7-9 after elastic recoil and including an enlarged view of a portion of the stent;
Figures 13-15 are Von Mises stress maps of the three stents of Figures 7-9 after application of a 0.2 MPa pressure to the external surface of the devices and including an enlarged view of a portion of the stent;
Figures 16-17 are Von Mises stress maps of the inner and outer stents of a 2PL stent model having 0 degrees of relative rotation and including an enlarged view of a portion of the stent;
Figures 18-19 are Von Mises stress maps of the inner and outer stents of a 2PL stent model having 22.5 degrees of relative rotation and including an enlarged view of a portion of the stent:
Figures 20-21 are Von Mises stress maps of the inner and outer stents of a $2PL_{1/2}$ stent model having 0 degrees of relative rotation and at 0.2MPa of pressure and including an enlarged view of a portion of the stent;
Figure 22 is a graph illustrating the radial displacement of several stents at their periphera section in response to an external pressure applied to emulate the compression force of the implantation site; and
Figure 23 is a graph illustrating the radial displacement of several stents at their middle section in response to an external pressure applied to emulate the compression force at the implantation site.

## Detailed Description

The properties of stents involved in the design of multi-layered stent constructions of the invention, which may be used for percutaneous pulmonary valve implantation, for example, desirably involve a compromise between interrelated and sometimes contradictory material and geometric properties of multiple stents. That is, the designs and materials selected for each of the stents of the multiple stent structures of the present invention are independently chosen to achieve certain desired overall performance characteristics for the stent. While the description and figures contained herein are primarily directed to two-layered stents, it is understood that multiple-layered stent structures having three or more stents are also contemplated by the invention, where some or all of the stents may be attached or connected in some way to at least one adjacent stent.

Referring now to the Figures, wherein the components are labeled with like numerals throughout the several Figures, and initially to Figures 4-6, three multiple stent structures 40, 50, 60 are illustrated, each of which generally comprises first and second stents 42, 44 (Figure 4), first and second stents 52, 54 (Figure 5), and first and second stents 62, 64 (Figure 6), respectively. The first and second stents of each of these embodiments are nested or positioned so that one stent is inside the other stent, and so that certain wires of the stents are differently offset relative to each other. In particular, first stent 42 of stent structure 40 is positioned within second stent 44 so that all or most of the wires of the first and second stents 42, 44 are generally adjacent to or aligned with each other (i.e., approximately 0 degrees of relative rotation). In other words, the stents 42, 44 are not offset or are only slightly offset relative to each other. First stent 52 of stent structure 50 is positioned within second stent 54, with the first stent 52 being rotated approximately 11.25 degrees relative to the second stent 54. First stent 62 of stent structure 60 is positioned within second stent 64, with the first stent 62 being rotated approximately 22.5 degrees relative to the second stent 64.

With any of the stent structures 40, 50, 60, their respective first and second stents may be attached or connected to each other in one or more locations where the wires of the stents are adjacent to and/or cross or overlap each other. Preferably, however, the number of attachment points or locations is selected to allow the first and second stents to flex or move somewhat independently of each other, which thereby provides certain advantages that can be achieved with the multi-layered stent structures of the invention and that are not necessarily attainable with only a single-layered structure. That is, the stents may be attached to each other at a predetermined number or percentage of possible attachment points, depending on the amount of potential relative movement that is anticipated. The stents may be attached, for example, at certain nodes near or at the inflow end of the stents and/or near or at the outflow end of the stents and/or at intermediate points along the length of the stents. It is noted that these same Figures 4-6 generally represent the structures used for the analysis performed below relative to the stents that are positioned within each other but that are not attached to each other. However, at least some of the principles of non-attached stents positioned within each other can also apply, at least generally, to stents that are attached to each other in a multi-layered stent structure. Alternatively, coverings on one or both stents could be attached to each other in addition to or in place of nodes.

The stents of a particular multi-layered stent structure can have the same lengths as each other, as shown, or may instead have somewhat or substantially different lengths. In addition, the diameters of the stents may be substantially identical to each other or may be different when unconstrained or uncompressed, although when they are configured with one or more stents positioned inside each other, as described herein, they desirably will have diameters that allow them to remain in contact with each other along all or most of their lengths. An inner stent is sufficiently self-expandable so that it will have roughly the same outer diameter as the inner diameter of the stent in which it is positioned. In this way, the two stents can maintain contact with each other after being implanted. The stents of the multi-layered stent structure can be generally centered about a common longitudinal axis that extends along the length of the stents such that at least a portion of the length of the stents can be considered to be concentrically or coaxially positioned relative to each other.

The individual stents of the multi-layered stent device of the invention are provided as discrete structures, where one discrete stent is positioned to be at least partially inside another discrete stent. That is, these stents cannot be considered to be a continuous braided structure arranged into more than one layer, but rather are independent structures arranged so that at least a portion of each of the stents of a single device are adjacent to or in contact with a portion of another stent of that device. Thus, each of the stents will have a first end that is at the opposite end of the stent from a second end, where these ends are spaced from each other along the length of the stent.

In one embodiment of the invention, each of the stents of a multi-layered stent structure can be attached to each other in a number of different ways, either prior to implantation or in a multiple step implantation process. If the stents are attached to each other prior to implantation, a number of different techniques and devices can be used, including welding, suturing, bending or folding of components or structures relative to each other, crimping, soldering, or other methods. Alternatively, features of each stent can be used for attachment to another stent, such as clips, barbs, hooks, rings, gaskets, clasps, magnets, or the like. In more general terms, the stents are configured to provide complimentary features that promote connection of the multiple stents. Alternatively, the stents may be attached to each other by friction or another type of attraction that does not involve physical connection of features or components of the stents. It is also contemplated that the stents can have more than one connection or attachment mechanism, and/or that each of the stents comprise different attachment features (e.g., one stent includes a barb and the other stent includes a magnet that attracts it to the other stent).

In another embodiment of the invention, the stents of a multi-layered structure are not attached to each other. However, with these structures, the individual stents are selected and/or designed to have certain properties that work in cooperation with properties of another stent or multiple stents that are selected and/or designed so that the overall structure has certain performance characteristics and/or features. In this way, materials and configurations of stents that are not particularly useful or desirable for a single stent is combined with another stent having different properties to achieve a combination stent structure desirable certain material properties.

The stents of a multi-layered stent structure of the invention are preferably made of materials and/or coatings selected to provide certain desirable properties to the structure, where certain properties may be more desirable for one of the stents in a structure than the others. For example, although platinum and iridium are mechanically somewhat weak materials, they also provide certain desirable characteristics to the percutaneous pulmonary valve implantation stents of the invention. That is, platinum-10% iridium alloy is biocompatible and has exceptional radiopacity due to its relatively high density as compared to some other materials (e.g., 21.55 g/cm$^3$ for the platinum-10% iridium alloy versus 7.95 g/cm$^3$ for stainless steel). The resulting high radio visibility allows for the use of relatively thin wires for the stent, which can result in improved flexibility and deliverability. In addition, the use of such a material for the designs of the stents of the invention allows for relatively easy crimping onto a balloon of a delivery system and allows for stent expansion at acceptable balloon pressures. The material further has a relatively small elastic recoil (e.g., <2%), which helps to provide a secure anchoring of the device at the implantation site. Thus, in cases where the properties exhibited by the platinum-iridium allow are desired for the stent structure, this alloy can be used for at least one stent of a multi-layered stent structure.

Other considerations that can be factored into the selection of materials and the design of the multi-layered stent include stent structural integrity over an extended time period (e.g., a certain number of months or years), the maintenance of radial strength, the integrity of the sutures between the valve and the stent, and the risk of embolization, such as in cases where little to no tissue growth occurs between the stent and tissue at the implantation location. Relatively high biocompatibility is also desirable to prevent thromboses and/or restenoses.

It is desirable for the stents of the multi-layered stent to be distinguishable from each other during and/or after implantation in a patient so that the clinician can determine certain performance characteristics of the stent and/or valve by independent evaluation of each of the stents, along with possible comparison of the performance of the stents to each other. This is accomplished with the use of different materials for the stents, such as providing stents having different radiopacity, echogenicity, and/or MRI signatures, for example.

The stents are preferably constructed of materials that are sufficiently flexible that they can be collapsed for percutaneous insertion into a patient. The material of the inner stent is self-expanding (e.g., Nitinol) in some embodiments, such that it can be readily compressed and reexpanded. The material should further be chosen so that when the stent system is positioned within an aorta, for example, one stent or a combination of stent structures exerts enough pressure against the aortic walls to prevent migration and minimize fluid leakage past the stent. In any of the embodiments of the invention, the replacement valves and associated stents can be provided in a variety of sizes to accommodate the size requirements of different patients. Materials that provide some or all of the properties described above can be selected for one or more of the stents of a multi-layered stent structure, in accordance with the invention.

The stents may be configured and constructed in a number of ways, where the configurations illustrated in the figures provide several exemplary constructions. The stents may be fabricated using wire stock or by machining the stent from a metal tube, as is sometimes employed in the manufacturing of stents. The number of wires, the positioning of such wires, and various other features of the stent can vary considerably from that shown in the figures. The specifics of the stent can vary widely within the scope of the invention, including the use of other cylindrical or cuff-like stent configurations. In any case, the stents are constructed so that the process of compressing the stent does not permanently deform the stent in such a way that expansion thereof would be difficult or impossible. That is, the stent should be capable of maintaining a desired structural integrity after being compressed and expanded.

In order to prevent possible interference between the patient's native valve and a replacement valve using a multiple stent structure, the native valve can be completely or partially removed. In some cases, the native valve may be left in its original location; however, the replacement valve in such a circumstance should be positioned in such a way that the remaining native valve does not interfere with its operation. In cases where the native valve is to be removed, exemplary valve removal or resection devices that can be used are described, for example, in PCT Publication WO/0308809A2.

In one exemplary delivery system for percutaneous pulmonary valve implantation, the multi-layered stent is loaded onto a delivery system that includes a deflated balloon, and the stent is crimped onto the balloon. The crimping can either be performed manually or with a crimping device or machine. The delivery system can then be inserted into the vascular environment, where the delivery system is manipulated within the anatomical pathways leading to the implantation site. The delivery of the stent to the desired location may be assisted by viewing the delivery process under fluoroscopy, for example.

In order to reach the desired location within the patient, such as the area of the aorta, the delivery system can be inserted into the body using one of a number of approaches. For example, the delivery device can reach the aorta through a retrograde approach originating at a location peripheral to the heart, such as the femoral artery. Alternatively, an antegrade approach could be used, which originates at a location peripheral to the heart, such as the femoral vein or an incision in the ventrical wall or apex. In any case, the *delivery device* is moved to the desired implantation area of the body with the multiple stent system optionally being partially or entirely enclosed within an outer sheath. The inner and outer stents can have different geometries and stiffnesses to allow for opening of stenosed, occluded, and/or improperly shaped vessels and/or valves.

Once the stent system is properly located within the patient, the outer stent can be deployed by gradually inflating the balloon, thereby expanding the stent to a desired size. Upon reaching a desired final stent diameter, the balloon can be deflated. Typically, such a removal of the radial pressure provided by the balloon will cause the stent to recoil or shrink to a somewhat smaller diameter, thus, the amount of anticipated recoil should be considered when inflating the balloon. In some cases, it will be desirable to pre-calculate the amount of anticipated recoil, based on the properties of the multiple stents, so that the proper amount of balloon expansion can be provided. That is, the stent can be expanded to a size that is larger than the desired final size so that it will shrink or recoil to the desired size when the radial force provided by the balloon is removed. The effects of both the outer and inner stents (or more stents, if such an embodiment is used) on each other relative to recoil can also be considered in the selection of the stents. That is, the use of two or more stents in a stent structure can influence the total amount of recoil encountered by the structure, which may be slightly or substantially different than the amount of recoil that would occur if the same stents were not attached to each other. In fact, many aspects of the multiple stents used can affect the recoil, including the materials and geometry of the stents, and also the type of attachment method used, along with other factors.

The amount of recoil can also be influenced by the pressure exerted by the implantation site wall, which can be measured, calculated, or estimated, depending on the circumstances. In any case, the expanded stent should be large enough in diameter that it places sufficient pressure on the vessel walls to prevent the device from becoming dislodged once it is implanted in the patient. In order to assess the performance of the device after its implantation, X-ray based imaging or other imaging techniques can be used to determine the location and condition of the multi-layered stent.

With any of the embodiments of the invention, a leaflet structure is attached within the mesh structure of the innermost stent, using any known attachment techniques, such as suturing. The stent structure can then be referred to as a valved stent, as the structure can then function as a valve when implanted in a patient. For such a structure, a polymer valve, tissue valve, or metal film valve can be used. In order to reduce the stitches required for valve attachment, a portion of the tissue can also or alternatively be trapped or positioned between stent layers. However, it is also possible that just the stent structures of the invention are implanted, with any corresponding leaflet structure omitted.

In order to analyze the performance of the multiple-layered stents of the invention, one or more stent structures that are believed to have the properties desired for a particular stent can be analyzed using finite element analyses. That is, the proposed multiple-layered stents can be examined with the stents being attached to each other in one of the manners described above and/or with the stents being positioned inside each other yet remaining unattached to each other. In either case, finite element analyses can be used to drive the engineering design process and prove the quality of a stent design before directly testing it in a patient. Parametric analyses enable prediction of the influence of some physical properties on the predicted mechanical behavior in order to optimize the final design of the device. That is, analyses of the type described herein can be used to determine certain characteristics of stents, and this information can be used for designing and/or selecting individual stents for use in a multi-layered stent assembly that has certain desired properties.

In one study performed on stents that were not attached to each other, large deformation analyses were performed using the finite element method (FEM) commercial code ABAQUS/Standard 6.4 (produced by ABAQUS, Inc., which was formerly known as Hibbit, Karlsson & Sorenses, Inc., of Pawtucket RI, USA), taking into account material and geometric nonlinearities. The use of a valve mounted into the stent was not considered for purposes of this study.

Three stent geometries were created on the basis of given data (e.g., from a supplier of the material) or obtained from measurements by means of calliper and optic microscope. The stent geometries were created to emulate the initial crimped status of a stent device onto a catheter balloon. The first model (herein referred to as the "PL stent") is illustrated generally in Figure 1 as stent 10 and is characterized by 6 zigzag wires formed into a tubular configuration, each having 8 crowns. The zigzag wires are arranged adjacent to each other along the same longitudinal axis so that crowns from adjacent wires are in contact with each other. The wires are welded together at these adjacent crowns. For this model, the diameter of the zigzag wires was 0.33 mm. The internal diameter of the stent was 4 mm and its overall length was 34.32 mm. The second model (herein referred to as the "PL-AU stent") had generally the same geometry as the first model, but further included gold brazed areas in the shape of 0.076 mm thick sleeves around the platinum wire crowns, such as is shown as sthent 20 in Figure 3. The third model (herein referred to as the "$PL_{1/2}$ stent") had the same design as the PL stent but with a wire diameter of 0.23 mm, which had a material mass that was half the mass of the PL stent.

A finite element model mesh was automatically generated. The stents were meshed with 10-node tetrahedrons in order to fit easily the complex geometries studied. The gold elements of the PL-AU model were tied to the platinum wires to avoid relative movement or separation between the two parts.

The stents used for this study were made of platinum-10% iridium alloy, for which the engineering stress-strain data for uni-axial tension tests includes a Young modulus of 224 GPa, a Poisson ratio of 0.37, and a yield stress of 285 MPa. The material behaves generally as a linear elastic solid up to the yield point. Beyond this point, time independent inelastic behavior was considered. The material was assumed to have isotropic properties. A Von Mises plasticity model, commonly used with metallic alloys, along with an isotropic hardening law was used in the analyses, as is described, for example, in the following articles: "Mechanical behavior modelling of balloon-expandable stents", Dumoulin et al., Journal of Biomechanics 2000; 33: 1461-1470; "Finite-element analysis of a stentotic revascularization through a stent insertion", Auricchio et al., Computer Methods and Biomechanics and Biomedical Engineering 2001; 4: 249-264; and "Stainless and shape memory alloy coronary stents: A computational study on the interaction with the vascular wall", Migliavacca et al., Biomechanics and Modeling in Mechanobiology 2004; 2(4): 205-217. Handbook properties were used for the mechanical behaviors of gold, including a Young modulus of 80 GPa, a Poisson ratio of 0.42, and a yield stress of 103 MPa.

Actual inflation of balloon-expandable stents in a clinical application is typically performed by pressurization of an elastomeric balloon inserted inside the device. However, because the intention of this study was to look at the stent in its final configuration (when the balloon was completely inflated) and after balloon deflation, the balloon was not modelled in the simulations.

Computationally, inflation of the stent may be performed using either direct pressure applied to the internal surface of the stent (load control) or through prescribed boundary conditions (displacement control). Attempts to expand the stent with direct pressure can prove difficult due to lack of geometrical symmetry in the design and could result in unrealistic deformations of the stent at the end of the expansion, as is described, for example, in the article "Finite element analysis and stent design: Reduction of dogboning", De Beule et al., Technology and Health Care 2006; 14 (4-5): 233-241. Consequently, the stent was inflated using radial expansion displacements up to an internal diameter of 24 mm (which is the maximum diameter reached by the device during actual percutaneous pulmonary valve implantation). Once the stent reached the desired diameter, the displacement constraints were removed to simulate the balloon deflation and allow the elastic recoil of the stent. Lastly, in order to simulate the compression force provided by the implantation site wall, a gradual pressure (load ramp) was applied to the external surface of the stent. This enabled evaluation of the stent strength to maintain the patency of the vessel.

To compare the performance of two coupled devices (stent-in-stent technique) against a single prosthesis, the inflation of two stents (with one positioned inside the other) was simulated. First, the outer stent was deployed up to 24 mm and released, as previously described. Next, the inner device was inflated up to 24 mm, thereby making contact with the outer stent. The displacement constraints were then removed to allow the stents to recoil. Finally, a pressure was applied to the external surface of the outer stent to evaluate the strength of the structure. The interaction between the two devices was described by a contact algorithm with friction, using a coefficient of sliding friction equal to 0.25.

The stent-in-stent analysis was performed with two PL stents (2PL) and two $PL_{1/2}$ stents ($2PL_{1/2}$). In particular, three different coupling configurations of the two PL stents were analyzed to assess the effect of the relative position between the inner and outer device: aligned (0 degrees) as in Figure 4, offset by 11.25 degrees of relative rotation as in Figure 5, and offset by 22.5 degrees of relative rotation as in Figure 6. For the $PL_{1/2}$ stent, only the aligned (0 degree) configuration as in Figure 4 was studied.

Before running the analyses, a sensitivity test was performed on the PL model mesh to achieve the best compromise between short calculation time and no influence of the element number on the results. In order to do this, five meshes with an increasing number of elements and nodes were tested, and the results are listed below in Table 1:

**Table 1 Mesh sensitivity analysis.**

|   | Spacing | Elements | Nodes | $R^{distal}$ [%] |
|---|---------|----------|-------|-----------|
| A | 0.17 | 85393 | 176424 | 1.58 |
| B | 0.15 | 95720 | 195365 | 1.57 |
| C | 0.12 | 166778 | 324518 | 1.55 |
| D | 0.115 | 218832 | 417126 | 1.55 |
| E | 0.1 | 284703 | 527852 | 1.54 |

For the analyses, the following mechanical properties were measured, calculated, and/or determined:

- Elastic recoil (R) following virtual balloon deflation in the stent middle ($R^{middle}$) and peripheral ($R^{peripheral}$) sections.

$$R = \frac{D_{load} - D_{unload}}{D_{load}} \cdot 100,$$

The elastic recoil is calculated as: with $D_{load}$ equal to the stent diameter at the end of the loading step and $D_{unload}$ equal to the stent diameter at the end of the unloading step. The difference in the elastic recoil ($\Delta R$) between peripheral and middle section of the stent was defined as: $\Delta R = R^{peripheral} - R^{middle}$.

- Von Mises stress ($\sigma_{VM}$) map at the end of virtual balloon inflation, deflation, and after application of the external pressure.
- Radial strength, represented by the plot of radial displacement resulting from the applied external pressure. The displacement was evaluated at both the peripheral and central nodes of the device.

Elastic recoil of the peripheral nodes of the stent and Von Mises stress color map were checked for the different meshes. The difference in elastic recoil between meshes decreased slightly with an increase in element number, as is shown in Table 1. The color map showed the same stress distribution for all meshes. The mesh which provided a solution independent from the mesh grid without a critical increase in calculation time was mesh C. The mesh of the gold parts, built around mesh C of the PL model, resulted in additional 116,602 elements for the PL-AU stent. The $PL_{1/2}$ mesh was made of 149,703 elements and 304,054 nodes.

Inflation by displacement control resulted in uniform radial expansion in all stent configurations. Upon balloon deflation, the elastic recoil (R) of the different devices was generally low, especially if compared to the values reported for stents used in different clinical indications. As expected, $R_{PL1/2}$ was larger than $R_{PL}$ because of the larger wire section of the PL stent, and $R_{PL}$ was greater than $R_{PL-AU}$ because of the gold reinforcement in the PL-AU stent, as is shown below in Table 2:

**Table 2 Elastic recoil values**

| Model | $R^{peripheral}$ [%] | $R^{middle}$ [%] | $\Delta R$ [%] |
|---|---|---|---|
| PL | 1.55 | 1.38 | 0.17 |
| PL-AU | 1.38 | 1.16 | 0.22 |
| $PL_{1/2}$ | 2.31 | 1.90 | 0.41 |
| 2PL - 0 degrees | 1.71 | 1.50 | 0.21 |
| 2PL - 11.25 degrees | 1.69 | 1.52 | 0.17 |
| 2PL - 22.5 degrees | 1.70 | 1.58 | 0.12 |
| $2PL_{1/2}$ - 0 degrees | 2.14 | 1.95 | 0.19 |

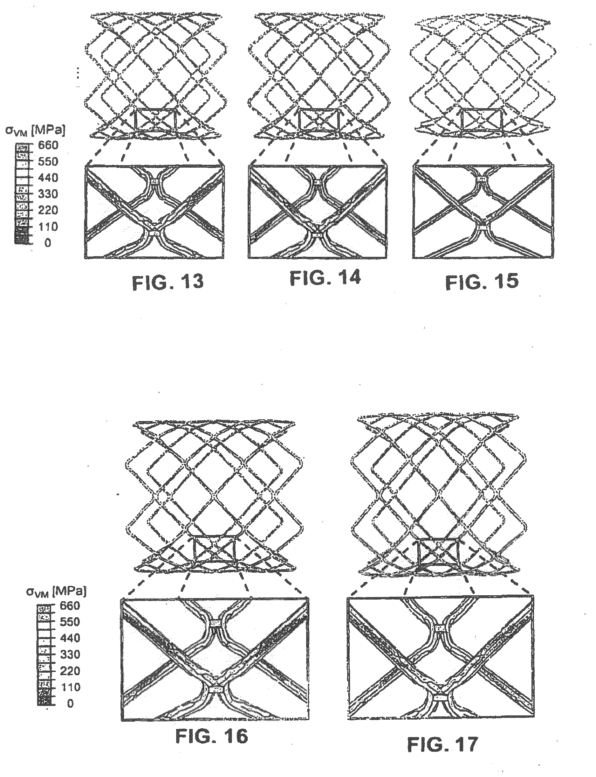

The difference in elastic recoil between the peripheral and middle sections was small for all of the stents. The highest $\Delta R$ was in the $PL_{1/2}$ stent, where the peripheral sections recovered more than the central part. Pressure applied uniformly to the external surface of the stent revealed that the peripheral sections of the $PL_{1/2}$ stent were also weaker than the central part in bolstering the arterial wall, as is shown in Figures 13-15.

The elastic recoil of the 2PL stent-in-stent analyses was almost the same in the three rotation configurations and $R_{PL}$ was less than $R_{2PL}$. For the same reason, $R^{middle}_{PL_{1/2}} < R^{middle}_{2PL_{1/2}}$. However, $R^{peripheral}_{PL_{1/2}} > R^{peripheral}_{2PL_{1/2}}$. Thus, the coupling of two $PL_{1/2}$ stents reinforced the peripheral sections of the structure.

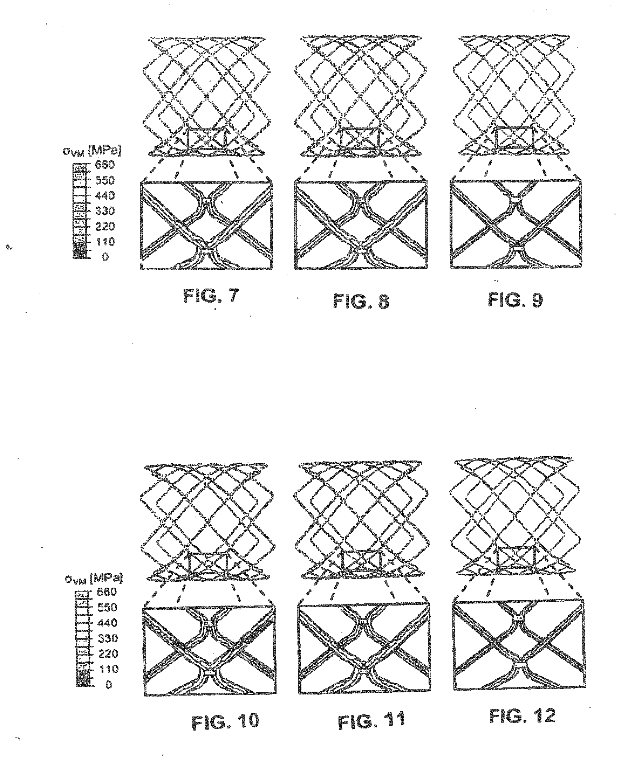

The Von Mises stress map at the inflated diameter of 24 mm is presented in Figures 7-9 for the PL, PL-AU and $PL_{1/2}$ stents, respectively. The highest stresses occurred in localized regions of the devices (i.e., at the strut intersections) where a peak of approximately 660 MPa was detected. Stress values throughout the stent were typically lower, diminishing rapidly from the crowns to the straight parts. These stresses were primarily due to the bending of the wires close to the platinum welds as the struts opened during inflation.

After virtual deflation of the balloon, at the end of the elastic recoil, as is shown in Figures 10-12, Von Mises stresses were lower everywhere due to the general unloading of the entire structure. When compared to the PL stent, the values of $\sigma_{VM}$ in PT-AU were slightly smaller, both at the end of the inflation step (Figures 7-9) and virtual balloon deflation (Figures 10-12). However, this difference was more evident when the external pressure was applied (see Figures 13-15), which signifies the situation when the stent has to resist the recovering force of the arterial wall.

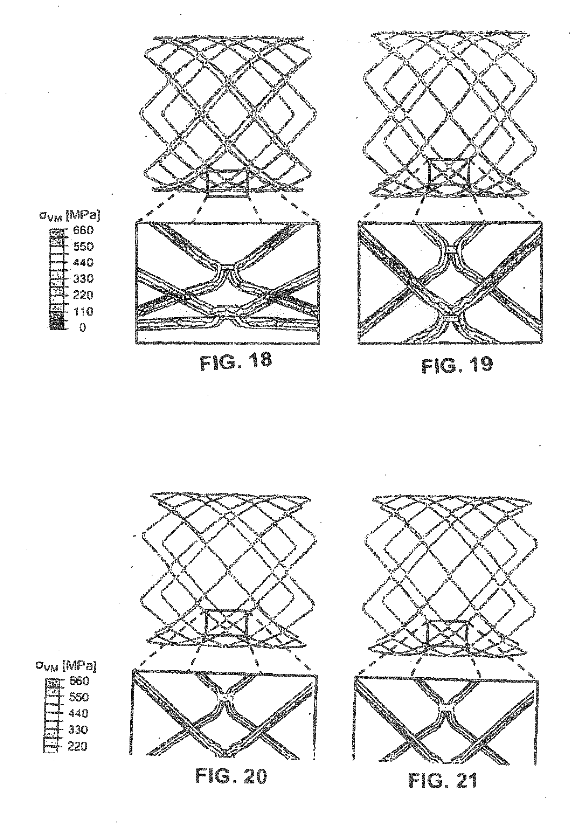

The 2PL model gave analogous results in terms of $\sigma_{VM}$ between the three different relative rotation couplings, as is depicted in Figures 16-19. The stress distribution in the inner 2PL stent was similar to that of the PL stent. However, the outer 2PL stent presented lower stress values than the PL device during the entire loading history. The same results were found for the $2PL_{1/2}$ inner and outer stents (as is shown in Figures 20-21) when compared to the $PL_{1/2}$ model.

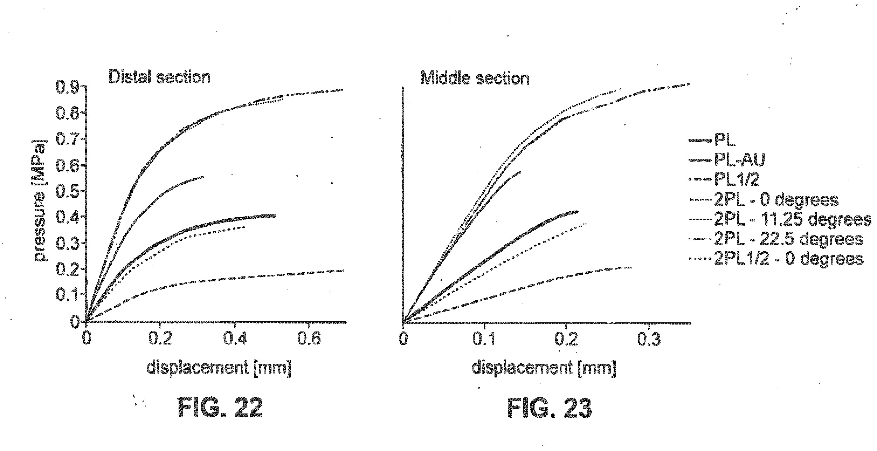

The charts of Figures 22-23 show the radial displacement of the peripheral and middle section nodes of the stents subject to external pressure. The trend lines are similar in the two sections for all devices. That is, at low pressure levels, high increases in pressure correspond to low displacements, as the devices possess adequate strength. However, as the pressure increases past a threshold, all structures lost their strength, and displacement increased disproportionately as compared to the pressure increases. The threshold pressure for each type of stent is different depending on its design.

As shown, the weaker device was the $PL_{1/2}$ stent, at least partially due to the thinner wire used to form it. The gold brazing of the PL-AU stent provided it with extra strength as compared to the non-reinforced PL stent. The relative rotation between the inner and outer stent in the 2PL devices did not influence the displacement response to the applied pressure. The 2PL model presented a higher strength than the single PL device and even than the PL-AU stent especially in the peripheral sections. The $2PL_{1/2}$ device was stronger than the single $PL_{1/2}$ stent and its strength was comparable to the PL stent.

This finite element study has shown that the maximum stresses reached in the device during inflation remained acceptable as compared to the platinum -10% iridium ultimate tensile strength of 875 Mpa provided by the manufacturer. However, the computational analyses indicate that the stress increases according to the expansion rate such that the safety of the device is highly dependent on the deployment magnitude.

The comparison between the PL and PL-AU models after external pressure application showed much lower stress in the PL-AU stent at the strut intersections. This is because in those points the resistant section of the PL-AU device is larger. The relatively weak gold actually reinforces the weld sections of the stent protecting them from fracture. However, it is possible to note a redistribution of the stress map in the straight platinum parts, at the end of gold reinforcements since the structure is loaded more in these points than without the reinforcement, because of the reinforcement itself.

The limited recent experience with the stent-in-stent technique demonstrate not only that repeat percutaneous pulmonary valve implantation is safe and feasible, but also that the implantation of a previous device before the valved one may be functional to bolster the vessel and ensure the integrity of the valved stent. The $2PL_{1/2}$ device compared to the PL stent showed the same ability to withstand the external pressure, the same stress distribution in the inner stent, but favorable lower stress values in the outer device. Because of its wire diameter, the two $PL_{1/2}$ stent employed in the $2PL_{1/2}$ model present the same material mass as the PL stent, but the thinner wire allows easier crimping, better deliverability and greater flexibility. The recoil is higher in the $2PL_{1/2}$ device than the PL stent. However, the finite element study showed that as the gold brazing reinforces the platinum wires, the elastic recoil is reduced. Therefore, a coupling of two PL-AU devices made of a thinner wire will provide better performances.

The pressure to compress the stents modeled in this study to a smaller diameter (see Figures 22-23) is relatively high if compared to certain data reported from mechanical tests in endovascular stents. In the finite element models, the pressure is uniformly applied along the stent circumference. In vitro tests, the device may be subjected to non-uniform loads. In-vivo, the stent conforms its shape to the implantation site. Some stent dimensions were assessed from angiographic pictures in the percutaneous pulmonary valve implantation patients. The measurements showed that the shape of the in-vivo stent differs from the theoretical cylindrical profile. Therefore, the force that the stent may be subjected to by the implantation site and the surrounding tissues are not uniform around the circumference. This can cause high-stress concentrations in some parts of the stents and increase the risk of fracture.

While the procedure described is directed to placement in the aortic annulus using a percutaneous catheter to deliver the valve retrograde to blood flow, antegrade delivery of the valve is also within the scope of the invention. Similarly, while delivery using a catheter is described, the valve could alternatively be compressed radially and delivered in a minimally invasive fashion using a tubular surgical trocar or port. In addition, the valve may be delivered to sites other than the aortic annulus. The multi-layered stent structures of the invention can utilize information provided from analyses of the type described above as a basis for selecting each of the stents of its structure, if desired, although the performance of the multiple stents when attached to each other can also be considered and analyzed in combination. Each of the stents of a multiple-layered stent structures can have the same or different geometries, as other stent(s) of the structure, and/or patterns and can be made of the same or different materials, where the stent structures utilize one or more of the attachment approaches of the invention. One or more of the stents of a multilayered stent structure can also include a covering, if desired, such as a polyethylene terephthalate (PET) material commercially available under the trade designation "Dacron", materials including a fluorine-containing polymer such as is commercially available under the trade designations "Teflon" and "Gore-Tex,", silicone, other biocompatible covering materials, or a combination of these and/or other materials that provide the desired properties. The coverings may be liquid impermeable or may be impermeable.

**Claims**

1. A radially compressible and expandable multi-layered stent assembly comprising:

   a first independent stent (42, 52, 62) comprising a first discrete end and a second discrete end;
   a second independent stent (44, 54, 64) comprising a first discrete end and a second discrete end and coaxially positioned within at least a portion of a length of the first stent (42, 52, 62); and
   a valve attached within an internal area of the second stent (44, 54, 64);

   wherein the first stent (42, 52, 62) comprises at least one material property that is different from at least one material property of the second stent (44, 54, 64),
   wherein a combination of a value of the at least one material property of the first stent (42, 52, 62) and a value of the at least one material property of the second stent (44, 54, 64) provides a combined material property value for the stent assembly that is different from the value of the material property of the first stent (42, 52, 62) and the value of the material property of the second stent (44, 54, 64),
   wherein the at least one material property of the first (42, 52, 62) and second (44, 54, 64) stents is flexibility, and
   wherein the first stent (42, 52, 62) has a lower flexibility than the second stent (44, 54, 64), and
   wherein the second stent (44, 54, 64) is a radially self-expanding stent.

2. The stent assembly of claim 1, wherein one of the first (42, 52, 62) and second (44, 54, 64) stents comprises at least one attachment feature that is attached to at least one attachment feature of the other of the first (42, 52, 62) and second (44, 54, 64) stents.

3. The stent assembly of claim 2, wherein the at least one attachment feature of at least one of the first (42, 52, 62) and second (44, 54, 64) stents comprises one of a clip, a barb, a hook, a ring, a gasket, a clasp, and a magnet.

4. The stent assembly of claim 1, wherein the first stent (42, 52, 62) is moveable relative to the second stent (44, 54, 64).

5. The stent assembly of claim 4, wherein the first stent (42, 52, 62) is longitudinally and/or radially moveable relative to the second stent (44, 54, 64).

6. The stent assembly of claim 1, wherein the first stent (42, 52, 62) is a balloon-expandable stent.

7. The stent assembly of claim 6, wherein the second stent (44, 54, 64) comprises a shape memory material.

8. The stent assembly of claim 1, wherein each of the first (42, 52, 62) and second (44, 54, 64) stents comprises a series of wire segments arranged in a tubular structure, wherein the wires segments of the first stent (42, 52, 62) are radially offset relative to the wire segments of the second stent (44, 54, 64) or wherein each of the wire segments of the first stent (42, 52, 62) are aligned with a corresponding wire segment of the second stent (44, 54, 64).

9. The stent assembly of claim 8, wherein the first stent (42, 52, 62) is rotated by one of 11.25 degrees or 22.5 degrees relative to the second stent (44, 54, 64).

10. The stent assembly of claim 1, wherein the multi-layered stent assembly comprises a third stent, wherein the first (42, 52, 62) and second (44, 54, 64) stents are coaxially positioned relative to the third stent.

11. The stent assembly of claim 1, wherein the valve comprises one of a polymer valve, a tissue valve, and a metal film valve.

12. The stent assembly of claim 1, wherein at least a portion of the valve is positioned between the first (42, 52, 62) and second stents (44, 54, 64).

13. The stent assembly of claim 1, wherein the first stent (42, 52, 62) and/or the second stent (44, 54, 64) comprise an outer covering layer.

14. The stent assembly of claim 1, wherein the at least one material property of the first (42, 52, 62) and second (44, 54, 64) stents is further wire density.

**Patentansprüche**

1.  Radial komprimierbare und expandierbare mehrschichtige Stent-Anordnung, umfassend:

    einen ersten unabhängigen Stent (42, 52, 62), umfassend ein erstes diskretes Ende und ein zweites diskretes Ende;
    einen zweiten unabhängigen Stent (44, 54, 64), umfassend ein erstes diskretes Ende und ein zweites diskretes Ende und der koaxial innerhalb mindestens eines Längenabschnitts des ersten Stents (42, 52, 62) positioniert ist; und
    eine Klappe, die in einem inneren Bereich des zweiten Stents (44, 54, 64) befestigt ist;
    wobei der erste Stent (42, 52, 62) mindestens eine Materialeigenschaft umfasst, die sich von mindestens einer Materialeigenschaft des zweiten Stents (44, 54, 64) unterscheidet, wobei eine Kombination aus einem Wert der mindestens einen Materialeigenschaft des ersten Stents (42, 52, 62) und einem Wert der mindestens einen Materialeigenschaft des zweiten Stents (44, 54, 64) einen kombinierten Materialeigenschaftswert für die Stent-Anordnung bereitstellt, der sich von dem Wert der Materialeigenschaft des ersten Stents (42, 52, 62) und dem Wert der Materialeigenschaft des zweiten Stents (44, 54, 64) unterscheidet, wobei die mindestens eine Materialeigenschaft des ersten (42, 52, 62) und zweiten (44, 54, 64) Stents Flexibilität ist, und wobei der erste Stent (42, 52, 62) eine geringere Flexibilität als der zweite Stent (44, 54, 64) aufweist, und wobei der zweite Stent (44, 54, 64) ein radial selbstexpandierender Stent ist.

2.  Stent-Anordnung nach Anspruch 1, wobei einer der ersten (42, 52, 62) und zweiten (44, 54, 64) Stents mindestens ein Befestigungsmerkmal umfasst, das an mindestens einem Befestigungsmerkmal des anderen der ersten (42, 52, 62) und zweiten (44, 54, 64) Stents befestigt ist.

3.  Stent-Anordnung nach Anspruch 2, wobei das mindestens eine Befestigungsmerkmal von mindestens einem der ersten (42, 52, 62) und zweiten (44, 54, 64) Stents eine Klammer, einen Widerhaken, einen Haken, einen Ring, eine Dichtung, eine Klammer und einen Magneten umfasst.

4.  Stent-Anordnung nach Anspruch 1, wobei der erste Stent (42, 52, 62) relativ zum zweiten Stent (44, 54, 64) beweglich ist.

5.  Stent-Anordnung nach Anspruch 4, wobei der erste Stent (42, 52, 62) in Längsrichtung und/oder radial relativ zum zweiten Stent (44, 54, 64) beweglich ist.

6.  Stent-Anordnung nach Anspruch 1, wobei der erste Stent (42, 52, 62) ein ballonexpandierbarer Stent ist.

7.  Stent-Anordnung nach Anspruch 6, wobei der zweite Stent (44, 54, 64) ein Formgedächtnismaterial umfasst.

8.  Stent-Anordnung nach Anspruch 1, wobei sowohl der erste (42, 52, 62) als auch der zweite (44, 54, 64) Stent eine Reihe von Drahtsegmenten umfasst, die in einer röhrenförmigen Struktur angeordnet sind, wobei die Drahtsegmente des ersten Stents (42, 52, 62) relativ zu den Drahtsegmenten des zweiten Stents (44, 54, 64) radial versetzt sind oder wobei jedes der Drahtsegmente des ersten Stents (42, 52, 62) mit einem entsprechenden Drahtsegment des zweiten Stents (44, 54, 64) ausgerichtet ist.

9.  Stent-Anordnung nach Anspruch 8, wobei der erste Stent (42, 52, 62) um 11,25 Grad oder 22,5 Grad relativ zum zweiten Stent (44, 54, 64) gedreht ist.

10. Stent-Anordnung nach Anspruch 1, wobei die mehrschichtige Stent-Anordnung einen dritten Stent umfasst, wobei der erste (42, 52, 62) und der zweite (44, 54, 64) Stent koaxial relativ zum dritten Stent positioniert sind.

11. Stent-Anordnung nach Anspruch 1, wobei die Klappe entweder eine Polymerklappe, eine Gewebeklappe oder eine Metallfilmklappe umfasst.

12. Stent-Anordnung nach Anspruch 1, wobei mindestens ein Abschnitt der Klappe zwischen dem ersten Stent (42, 52, 62) und dem zweiten Stent (44, 54, 64) positioniert ist.

13. Stent-Anordnung nach Anspruch 1, wobei der erste Stent (42, 52, 62) und/oder der zweite Stent (44, 54, 64) eine äußere Deckschicht umfasst.

**14.** Stent-Anordnung nach Anspruch 1, wobei die mindestens eine Materialeigenschaft des ersten (42, 52, 62) und zweiten (44, 54, 64) Stents ferner die Drahtdichte ist.

**Revendications**

**1.** Ensemble de stent multicouche compressible et extensible radialement comprenant :

un premier stent indépendant (42, 52, 62) comprenant une première extrémité discrète et une seconde extrémité discrète ;
un deuxième stent indépendant (44, 54, 64) comprenant une première extrémité discrète et une seconde extrémité discrète et étant positionné coaxialement à l'intérieur d'au moins une partie d'une longueur du premier stent (42, 52, 62) ; et
une valve fixée à l'intérieur d'une zone interne du deuxième stent (44, 54, 64) ;
dans lequel le premier stent (42, 52, 62) comprend au moins une propriété de matériau qui est différente d'au moins une propriété de matériau du deuxième stent (44, 54, 64),
dans lequel une combinaison d'une valeur de l'au moins une propriété de matériau du premier stent (42, 52, 62) et une valeur de l'au moins une propriété de matériau du deuxième stent (44, 54, 64) fournit une valeur de propriété de matériau combinée pour l'ensemble de stent qui est différente de la valeur de la propriété de matériau du premier stent (42, 52, 62) et de la valeur de la propriété de matériau du deuxième stent (44, 54, 64),
dans lequel l'au moins une propriété de matériau du premier (42, 52, 62) et du deuxième (44, 54, 64) stents est une flexibilité, et dans lequel le premier stent (42, 52, 62) a une flexibilité inférieure à celle du deuxième stent (44, 54, 64), et
dans lequel le deuxième stent (44, 54, 64) est un stent auto-expansible radialement.

**2.** Ensemble de stent selon la revendication 1, dans lequel l'un des premier (42, 52, 62) et deuxième (44, 54, 64) stents comprend au moins une caractéristique de fixation qui est fixée à au moins une caractéristique de fixation de l'autre du premier (42, 52, 62) et deuxième (44, 54, 64) stents.

**3.** Assemblage de stent selon la revendication 2, dans lequel l'au moins une caractéristique de fixation d'au moins l'un des premier (42, 52, 62) et deuxième (44, 54, 64) stents comprend l'un quelconque parmi un clip, un ardillon, un crochet, une bague, un joint, un fermoir et un aimant.

**4.** Ensemble de stent selon la revendication 1, dans lequel le premier stent (42, 52, 62) est mobile par rapport au deuxième stent (44, 54, 64).

**5.** Ensemble de stent selon la revendication 4, dans lequel le premier stent (42, 52, 62) est mobile longitudinalement et/ou radialement par rapport au deuxième stent (44, 54, 64).

**6.** Ensemble de stent selon la revendication 1, dans lequel le premier stent (42, 52, 62) est un stent expansible par ballonnet.

**7.** Ensemble de stent selon la revendication 6, dans lequel le deuxième stent (44, 54, 64) comprend un matériau à mémoire de forme.

**8.** Ensemble de stent selon la revendication 1, dans lequel chacun des premier (42, 52, 62) et deuxième (44, 54, 64) stents comprend une série de segments de fil disposés en une structure tubulaire, dans lequel les segments de fil du premier stent (42, 52, 62) sont décalés radialement par rapport aux segments de fil du deuxième stent (44, 54, 64) ou dans lequel chacun des segments de fil du premier stent (42, 52, 62) sont alignés avec un segment de fil correspondant du deuxième stent (44, 54, 64).

**9.** Ensemble de stent selon la revendication 8, dans lequel le premier stent (42, 52, 62) est tourné de l'un quelconque parmi 11,25 degrés ou 22,5 degrés par rapport au deuxième stent (44, 54, 64).

**10.** Ensemble de stent selon la revendication 1, dans lequel l'ensemble de stent multicouche comprend un troisième stent, dans lequel les premier (42, 52, 62) et deuxième (44, 54, 64) stents sont positionnés coaxialement par rapport au troisième stent.

**11.** Ensemble de stent selon la revendication 1, dans lequel la valve comprend l'une quelconque parmi une valve en polymère, une valve en tissu et une valve en film métallique.

**12.** Ensemble de stent selon la revendication 1, dans lequel au moins une partie de la valve est positionnée entre les premier (42, 52, 62) et deuxième stents (44, 54, 64).

**13.** Ensemble de stent selon la revendication 1, dans lequel le premier stent (42, 52, 62) et/ou le deuxième stent (44, 54, 64) comprennent une couche de revêtement externe.

**14.** Ensemble de stent selon la revendication 1, dans lequel l'au moins une propriété de matériau des premier (42, 52, 62) et deuxième (44, 54, 64) stents est une densité de fil supplémentaire.

**Fig. 1**

**Fig. 2**

**Fig. 3**

**Fig. 4**

**Fig. 5**

**Fig. 6**

σ_VM [MPa]
660
550
440
330
220
110
0

**FIG. 7**          **FIG. 8**          **FIG. 9**

σ_VM [MPa]
660
550
440
330
220
110
0

**FIG. 10**          **FIG. 11**          **FIG. 12**

$\sigma_{VM}$ [MPa]

660
550
440
330
220
110
0

FIG. 13

FIG. 14

FIG. 15

$\sigma_{VM}$ [MPa]

660
550
440
330
220
110
0

FIG. 16

FIG. 17

$\sigma_{VM}$ [MPa]

660
550
440
330
220
110
0

FIG. 18

FIG. 19

$\sigma_{VM}$ [MPa]

660
550
440
330
220

FIG. 20

FIG. 21

FIG. 22

FIG. 23

## REFERENCES CITED IN THE DESCRIPTION

**Patent documents cited in the description**

- US 20050251251
- US 20030199971 A1
- US 20030199963 A1
- US 6425916 B, Garrison
- US 7060089 B, Ley
- US 20050075725, Rowe
- US 20050251251 A, Cribier
- US 20060271166 A, Thill
- US 20060276874 A, Wilson
- US 20070213813 A, Von Segesser
- WO 2007053243 A, Salahieh
- WO 2006054107 A, Bonhoeffer
- WO 2007081820 A, Nugent
- WO 0308809 A2

**Non-patent literature cited in the description**

- **BONHOEFFER et al.** Percutaneous insertion of the pulmonary valve. *Journal of the American College of Cardiology,* 2002, vol. 39 (10), 1664-1669
- **BONHOEFFER et al.** Transcatheter Replacement of a Bovine Valve in Pulmonary Position. *Circulation,* 2000, vol. 102, 813-816
- **BONHOEFFER et al.** Percutaneous replacement of pulmonary valve in a right-ventricle to pulmonary-artery prosthetic conduit with valve dysfunction. *Lancet,* 2000, vol. 356 (9239), 1403-1405
- **KHAMBADKONE et al.** Percutaneous pulmonary valve implantation in humans: results in 59 consecutive patients. *Circulation,* 2005, vol. 112 (8), 1189-1197
- **COATS et al.** Physiological and clinical consequences of relief of right ventricular outflow tract obstruction late after repair of congenital heart defects. *Circulation,* 2006, vol. 113 (17), 2037-2044
- **POWELL et al.** Prolongation of RV-PA conduit life span by percutaneous stent implantation. Intermediate Term Results. *Circulation,* 1995, vol. 92 (11), 3282-3288
- **KNIRSCH et al.** Longitudinal stent fracture 11 months after implantation in the left pulmonary artery and successful management by a stent-in-stent maneuver. *Catheterization and Cardiovascular Interventions,* 2003, vol. 58, 116-118
- **SUGIYAMA et al.** Implantation of endovascular stents for the obstructive right ventricular outflow tract. *Heart,* 2005, vol. 91 (8), 1058-1063
- **CARROZZA et al.** Stress stent fracture: Is stent angioplasty really a safe therapeutic option in native aortic coarctation?. *International Journal of Cardiology,* 2006, vol. 113 (1), 127-128
- **DUMOULIN et al.** Mechanical behavior modelling of balloon-expandable stents. *Journal of Biomechanics,* 2000, vol. 33, 1461-1470
- **AURICCHIO et al.** Finite-element analysis of a stenotic revascularization through a stent insertion. *Computer Methods and Biomechanics and Biomedical Engineering,* 2001, vol. 4, 249-264
- **MIGLIAVACCA et al.** Stainless and shape memory alloy coronary stents: A computational study on the interaction with the vascular wall. *Biomechanics and Modeling in Mechanobiology,* 2004, vol. 2 (4), 205-217
- **DE BEULE et al.** Finite element analysis and stent design: Reduction of dogboning. *Technology and Health Care,* 2006, vol. 14 (4-5), 233-241